# EUROPEAN PATENT SPECIFICATION

(11) **EP 4 064 466 B1**
(45) Date of publication and mention of the grant of the patent: **18.02.2026**
(21) Application number: 21904616.6
(22) Date of filing: 08.04.2021
(51) Int. Cl.: A61H 1/00, H01R 25/14, G16H 20/30, A61H 7/00

(54) **FLEXIBLE SLIDING POWER SUPPLY ASSEMBLY, MASSAGE CUSHION, AND CONTROL METHOD THEREFOR**
FLEXIBLE SCHIEBEENERGIEVERSORGUNGSANORDNUNG SOWIE MASSAGEKISSEN UND STEUERVERFAHREN DAFÜR
ENSEMBLE D'ALIMENTATION COULISSANT FLEXIBLE, COUSSIN DE MASSAGE, ET PROCÉDÉ DE COMMANDE CORRESPONDANT

(30) Priority: 17.12.2020 CN 202011496068
(43) Date of publication of application: 28.09.2022
(73) Proprietor: Shenzhen Mondial Technology Co., Limited, Shenzhen, Guangdong 518116 (CN)
(72) Inventor: REN, Changhui, Shenzhen Guangdong 518116 (CN)
(74) Representative: Cleanthous, Marinos
(86) International application number: PCT/CN2021/000073
(87) International publication number: WO 2022/126798

(56) References cited:
- CN-A- 110 836 337
- CN-A- 112 510 458
- CN-U- 209 611 675
- CN-U- 209 611 675
- CN-U- 209 827 440
- DE-A1- 102015 001 154
- DE-A1- 102016 204 103
- US-A- 5 561 271
- US-A1- 2005 245 851

## Description

### TECHNICAL FIELD

The present disclosure relates to the field of massagers, specifically to a flexible sliding powers supply assembly, and a massage cushion and a control method therefor.

### BACKGROUND

A massager belongs to a kind of conventional health care equipment. The massager has several soft-touch massage heads, which can relax muscles, soothe nerves, promote blood circulation, relieve fatigue, significantly reduce various chronic pains, acute pains and muscle soreness, relax the body and reduce stress. As a massager, a massage cushion is convenient to install and use, and is widely used in automobiles seats, office chairs, or sofas.

With the improvement of people's living standards, demands for the massage cushion in the market are more and more diversified. In order to make the massage cushion suitable for massage on various kinds of seats, the massage cushions are increasingly required to be light and thin. When a traditional massage movement walks, since a spring-shape wire has a too large diameter, it is impossible to make the massage cushion thinner while conforming to the human curve.

If a special mold is used to improve the problem that a fixed conductive rail occupies a large space, the massage cushion needs to be customized according to the shape of a seat, so that the development cost is high and the development period is long. When the whole is thinner, a guide rail may be prone to problems such as intermittent poor contact and derailment.

At the same time, in order to massage multiple areas at the same time, the existing massage cushion is usually provided with multiple groups of movements. When a power supply main board is controlled to be connected to power or communicate with the multiple groups of movements, each connected movement is connected by one wire, resulting in that more movements require move wires, so that the machining difficulty is high, and the assembling and maintenance cost is high.

Up-and-down action of the massage movement of the existing massage cushion in the prior art mainly relies on the following three conductive connection manners:

The first manner is wire connection. A wire can be bent to be folded and unfolded. With the up-and-down action of the massage movement, since a moving trajectory of the wire cannot be fixed, the massage movement cannot effectively avoid a position where a person can squeeze, so that after the wire is squeezed by an external force and the person, the moving trajectory will change, or the wire cannot be bent and unfolded, which makes the wire broken or twined on a massage head. As a result, a product malfunctions, and frequent folding and unfolding of the wire will cause a copper wire inside the wire to be broken.

The second manner is spring-shape wire connection. However, since a spring-shape wire is spiral, the diameter of the wire will increase. When moving on a thinner cushion shell, the spring-shape wire will be easily squeezed by a person, causing a malfunction. Furthermore, if the spring-shape wire is frequently stretched and bent, a copper wire in it will also be broken.

The third manner is fixed conductive guide rail connection. In this conductive connection method, special conductive guide rails need to be customized and designed for each kind of massage chairs with different shapes, and a special mold needs to be opened. Furthermore, a fixed conductive guide rail occupies a large space, has high development cost, and long development period. When the massage movement moves or there is debris on the guide rail, the problems of poor intermittent contact, derailment, and the like occur.

There are also massage chairs that use electrified guide rails, such as a novel conductive structure of a massage chair movement ZL201820665485.X, a guide rail of which is of a rigid structure and cannot be used for massagers with certain flexibility, such as a massage cushion. Meanwhile, due to the rigid structure, in order to satisfy the use of different types of equipment, customized molds need to be used, so the cost will increase significantly.

CN209611675 U discloses a novel conductive structure of a massage armchair movement. The novel conductive structure comprises a movement, the movement is provided with a conductive assembly which is electrically connected with the control circuit. The conductive assembly comprises a fixed guide rail and a position adjusting guide groove moving together with the movement. The position adjusting guide groove is connected to the guide rail in a sleeving mode. And the conducting strip arranged on the guide rail is continuously and electrically connected with the conductive contact of the position adjusting guide groove.

US2005245851 A1 discloses a body massager comprising a portable housing including a backrest and a seat support. A longitudinal guide is provided in the backrest cooperating with a carriage for translation of the carriage within the backrest and a motor drives the carriage along the guide. A pair of massage members are supported by the carriage and extend from the backrest for imparting a massage effect upon the back of the user. The seat support includes a massager therein for imparting another massage effect to the user.

### SUMMARY

For the above problems, the present disclosure aims to provide a flexible sliding power supply assembly with stable structure, good contact and low manufacturing cost, and a massage cushion and a control method therefor.

In order to achieve the technical objective, a solution of the present disclosure is as follows: A flexible sliding power supply assembly includes a flexible conductive rail and a sliding conductive assembly. The flexible conductive rail is provided with three fixing slots, and three conductive bars are mounted in the fixing slots in an embedded manner. Two conductive bars are used for supplying power, and the third conductive bar is used for controlling signals; the conductive bars are electrically connected with a controller.

The sliding conductive assembly is mounted on the flexible conductive rail; the sliding conductive assembly is in slidably electric connection with the conductive bars; and the conductive bars are correspondingly electrically connected to a power supply and a controller. The circuit board is electrically connected with a micro control unit (MCU).

The sliding conductive assembly includes a probe control sleeve, conductive probes, and a circuit board; the circuit board is mounted in the probe control sleeve; and the circuit board is electrically connected with a plurality of conductive probes.

The middle part of the probe control sleeve is a connecting opening; by the connecting opening, the probe control sleeve is capable of being mounted on the flexible conductive rail; and the conductive probes protrude from the connecting opening and are electrically connected to the conductive bars on the flexible conductive rail.

As a preference, one side of the probe control sleeve is provided with a connecting part fixedly connected to a massage movement; and each conductive bar is correspondingly electrically connected to one or more conductive probes.

Each conductive probe is formed by combining a copper pin, a spring and a wire.

As a preference, one or more magnets are mounted on a flexible conductive rail in an embedded manner; a hall switch is mounted on the circuit board of the massage movement; and the hall switch can determine the position of the movement by sensing a magnetic field formed by the magnets.

As a preference, the fixing slots are " 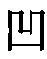 "-shaped; each fixing slot includes a fixed part and an opening part; a width and thickness of the fixed part are both equal to those of each conductive bar; a width of the opening part is less than that of the conductive bar; and a thickness of the opening part is less than an extending height of the conductive probe.

A massage cushion adopts the flexible sliding power supply assembly. The flexible conductive rail is fixedly mounted on a cushion body; the other side of the cushion body is further provided with a rack; and one or more massage movements are mounted between the flexible conductive rail and the rack.

One side of each massage movement is meshed with the rack; the other side of the massage movement is fixedly provided with the sliding conductive assembly; and the massage movement is electrically connected with each conductive bar through the sliding conductive assembly.

As a preference, the fixing slots of the flexible conductive rail are inwardly disposed; the circuit board of the probe control sleeve and one side of each conductive probe is inwardly disposed; and a massage head on each massage movement is outwardly disposed.

An upper surface and a lower surface of the probe control sleeve are both of arc curved structures.

A control method is applied to the massage cushion. The control method specifically includes the following steps:
S1, control: issuing, by means of a mobile phone or the controller, an instruction; encoding the instruction through a communication protocol; transmitting the encoded instruction to the MCU via a specified conductive bar; and decoding, by the MCU, the encoded instruction to control the massage movement to perform a specified action; and
S2, feedback: when the massage movement switches a mode or stops working, encoding, by the MCU, a state; transmitting the encoded state to the controller via a specified conductive bar; and decoding, by a central processing unit (CPU) on the controller, the encoded state to obtain a working state of the massage movement.

As a preference, the control method further includes step S3, positioning: sensing, by the MCU, a magnet on a flexible conductive rail of the cushion body through the hall switch on the circuit board; after the MCU determines the position of the massage movement, encoding, by the MCU, position information; transmitting the encoded position information to the controller via a specified conductive bar; and decoding, by the CPU on the controller, the encoded position information to obtain the position information of the massage movement.

The present disclosure has the beneficial effects. The flexible conductive rail in the sliding power supply assembly of the present disclosure is made of high-toughness engineering plastic. The flexible conductive rail can bend in a large arc in one direction to adapt to massage chairs with different shapes, so that the adaptability is good, and it can be used for both a common cushion and a curved cushion. In the flexible conductive rail, the tunnel type sliding conductive assembly can be made to be a standard component, thus adapting to various kinds of cushions, reducing the development cost, facilitating the assembling, reducing the kinds of materials, and decreasing the product cost. The flexible conductive rail is connected by the tunnel type probe control sleeve structure, so that reliable fixing is achieved during sliding guidance, and the problem of derailment is completely solved. Each conductive bar corresponds to two conductive probes. The parallel conductive probes have good contact-conducting stability, so that the problem of poor contact easily caused in the existing fixed conductive rail is solved. In the flexible conductive rail of the present disclosure, one controller can be connected to a plurality of massage movements with the tunnel type sliding conductive assemblies by only three conductive bars, so that the number of wires is reduced, and the problems of breakage of wires of the massage cushion and twining of the wires on a massage head are completely solved. The flexible conductive rail has a normative appearance and a compact structure, and the moving trajectory can be fixed according to the movement. The sliding conductive assembly is located on a side surface. One protruding end of the circuit board of the sliding conductive assembly is located on an inner side, and at the same time, the upper and lower end parts of the sliding conductive assembly are both arc-shaped, so that a position where a person easily squeezes can be bypassed; a product has more stable quality; and the person feels more comfortable during massage.

### BRIEF DESCRIPTION OF THE DRAWINGS

FIG. 1 is a schematic structural diagram of a sliding conductive assembly of the present disclosure;
FIG. 2 is a sectional view of a sliding conductive assembly of the present disclosure;
FIG. 3 is an exploded diagram of a sliding conductive assembly of the present disclosure;
FIG. 4 is a schematic structural diagram of the present disclosure;
FIG. 5 is an exploded diagram of a flexible conductive rail of the present disclosure;
FIG. 6 is a schematic structural diagram of a massage cushion of the present disclosure.

### DETAILED DESCRIPTION OF THE EMBODIMENTS

The present disclosure is further described in detail below in combination with the accompanying drawings and specific embodiments.

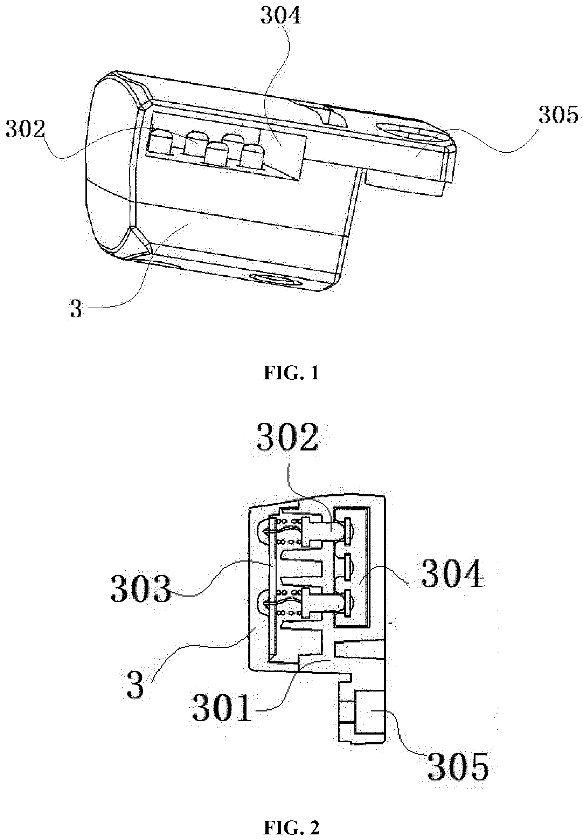

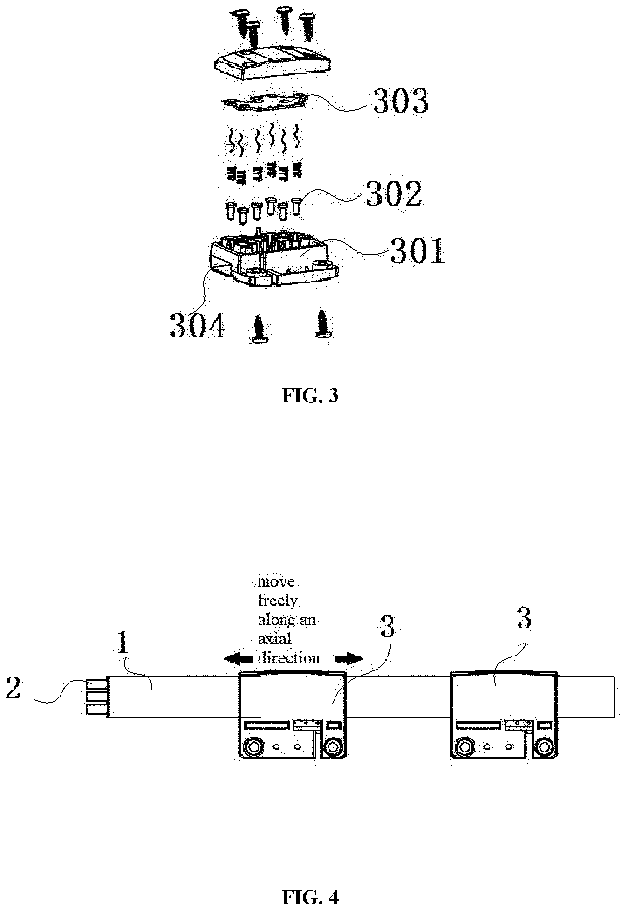

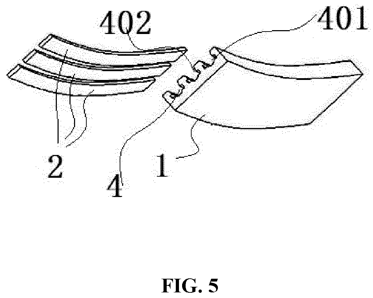

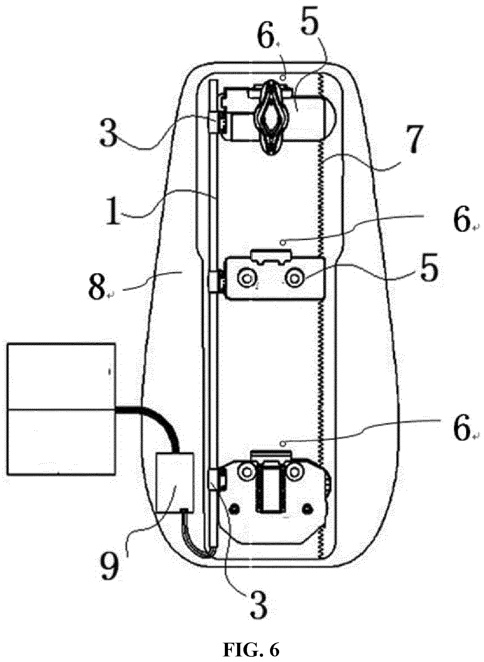

As shown in FIGS. 1-6, a specific embodiment of the present disclosure is a flexible sliding power supply assembly, including a flexible conductive rail 1 and a sliding conductive assembly 3. The flexible conductive rail 1 is provided with two or more fixing slots 4, and conductive bars 2 are mounted in the fixing slots 4 in an embedded manner.

A sliding conductive assembly 3 is mounted on the flexible conductive rail 1; the sliding conductive assembly 3 is in slidably electric connection with the conductive bars 2; and the conductive bars 2 are correspondingly electrically connected to a power supply and a controller 9.

The sliding conductive assembly 3 includes a probe control sleeve 301, conductive probes 302, and a circuit board 303; the circuit board 303 is mounted in the probe control sleeve 301; and the circuit board 303 is electrically connected with a plurality of conductive probes 302. The probe control sleeve of the present disclosure is of a tunnel type structure. The tunnel type probe control sleeve can ensure that an assembly will not be loosened and fall off from the flexible conductive rail and can also ensure that the conductive probes are always kept in a stable electric connection state during vibration. During use, under squeezing, the probe control sleeve can also move with the flexible conductive rail to keep the stable electric connection. The traditional conductive rail adopts a rigid rail, which cannot adapt to a vibration environment of a massager and a squeezed usage scenario.

The middle part of the probe control sleeve 301 is a connecting opening 304; by the connecting opening 304, the probe control sleeve can be mounted on the flexible conductive rail 1; and the conductive probes 302 protrude from the connecting opening 304 and are electrically connected to the conductive bars 2 on the flexible conductive rail 1.

One side of the probe control sleeve 301 is provided with a connecting part 305 fixedly connected to a massage movement 5; and each conductive bar 2 is correspondingly electrically connected to one or more conductive probes 302. Each conductive probe 302 is formed by combining a copper pin, a wire and a spring. Meanwhile, a plurality of conductive probes can enlarge a contact area, which can meet a requirement for high current.

One or more magnets 6 are mounted on an operating rail of the massage movement 5 on a back shell of a cushion body in an embedded manner; a hall switch is mounted on the circuit board of the massage movement 5; and the hall switch can determine the position of the movement by sensing a magnetic field formed by the magnets 6.

The fixing slots 4 are " 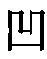 "-shaped; each fixing slot 4 includes a fixed part 401 and an opening part 402; a width and thickness of the fixed part 401 are both equal to those of each conductive bar 2; a width of the opening part 402 is less than that of the conductive bar 2; and a thickness of the opening part 402 is less than an extending height of the copper pin.

A massage cushion adopts the flexible sliding power supply assembly. The flexible conductive rail is fixedly mounted on a cushion body 8; the other side of the cushion body 8 is further provided with a rack 7; and one or more massage movements 5 are mounted between the flexible conductive rail 1 and the rack 7.

One side of each massage movement 5 is meshed with the rack 7; the other side of the massage movement 5 is fixedly provided with the sliding assembly 3; and the massage movement 5 is electrically connected with each conductive bar through the sliding conductive assembly 3.

The fixing slots 4 of the flexible conductive rail 1 are inwardly disposed; the circuit board 303 of the probe control sleeve 301 and one side of each conductive probe 302 is inwardly disposed; and a massage head on each massage movement 5 is outwardly disposed.

An upper surface and a lower surface of the probe control sleeve 301 are both of arc curved structures.

A control method is applied to a massage cushion. The flexible conductive rail is provided with three conductive bars; two conductive bars are used for supplying power, and the third conductive bar is used for controlling signals; the conductive bars are also electrically connected with a controller; the circuit board is also electrically connected with an MCU; and the control method specifically includes the following steps:
S1, control: issuing, by means of a mobile phone or the controller, an instruction; encoding the instruction through a communication protocol; transmitting the encoded instruction to the MCU via a specified conductive bar; and decoding, by the MCU, the encoded instruction to control the massage movement to perform a specified action; and
S2, feedback: when the massage movement switches a mode or stops working, encoding, by the MCU, a state; transmitting the encoded state to the controller via a specified conductive bar; and decoding, by a central processing unit (CPU) on the controller, the encoded state to obtain a working state of the massage movement.

The control method further includes step S3, positioning: sensing, by the MCU, a magnet on a back shell rail of the cushion body through the hall switch on the circuit board; after the MCU determines the position of the massage movement, encoding, by the MCU, position information; transmitting the encoded position information to the controller via a specified conductive bar; and decoding, by the CPU on the controller, the encoded position information to obtain the position information of the massage movement.

When the movement of the traditional massage cushion walks up and down to achieve massage, a connecting wire is easy to break due to a bend. The following problems are solved: When the movement of the massage cushion walks up and down to achieve massage, since the connecting spring-shape wire has a large diameter, the diameter of the spring-shape wire is extremely large during making of an ultrathin structure, and a person will squeeze the spring-shape wire when the person leans against the massage cushion. As a result, the existing fixed conductive guide rail is pulled apart since the wire is not extensible, and the adaptability is poor. Special conductive guide rails are required to be designed for use for different massage cushions, and special molds need to be opened to manufacture the conductive guide rails. An existing guide rail conduction device is fixed in a pressed manner. The conduction device is easy to jump when moving along a conductive rail, resulting in poor intermittent contact, affecting power supplying and signal transmission, and causing part of signals to be lost. The pressed fixed guide rail has the risk of derailment. For example, in a novel conductive structure of a massage chair movement ZL201820665485.X, its conductive assembly cannot be applied to a flexible conductive rail. If the conductive assembly is forcibly combined with the flexible conductive rail, the conductive assembly is squeezed to easily cause derailment or poor contact. The existing guide rail adopts a single carbon brush (or conductive pillar) to contact a conductive metal bar on the guide rail to achieve electricity or signal transmission. When there is debris such as dust on the conductive bar, the conduction device is easy to jump, resulting in poor intermittent contact, which affects the power supplying and signal transmission and causes part of signals to be lost.

The flexible conductive rail of the present disclosure is made of high-toughness engineering plastic. The flexible conductive rail can bend in a large arc in one direction to adapt to massage chairs with different shapes, so that the adaptability is good, and it can be used for both a common cushion and a curved cushion. In the flexible conductive rail, the tunnel type sliding conductive assembly can be made to be a standard component, thus adapting to various kinds of cushions, reducing the development cost, facilitating the assembling, reducing the kinds of materials, and decreasing the product cost. The flexible conductive rail is connected by the tunnel type probe control sleeve structure, so that reliable fixing is achieved during sliding guidance, and the problem of derailment is completely solved. Each conductive bar corresponds to two conductive probes. The parallel conductive probes have good contact-conducting stability, so that the problem of poor contact easily caused in the existing fixed conductive rail is solved. In the flexible conductive rail of the present disclosure, one controller can be connected to a plurality of massage movements with the tunnel type sliding conductive assemblies by only three conductive bars, so that the number of wires is reduced, and the problems of breakage of wires of the massage cushion and twining of the wires on a massage head are completely solved. The flexible conductive rail has a normative appearance and a compact structure, and the moving trajectory can be fixed according to the movement. The sliding conductive assembly is located on a side surface. One protruding end of the circuit board of the sliding conductive assembly is located on an inner side, and at the same time, the upper and lower end parts of the sliding conductive assembly are both arc-shaped, so that a position where a person easily squeezes can be bypassed; a product has more stable quality; and the person feels more comfortable during massage.

## Claims

1. A flexible sliding power supply assembly, comprising a conductive rail (1) and a sliding conductive assembly (3), wherein the conductive rail (1) is provided with three fixing slots (4); three conductive bars (2) are mounted in the fixing slots (4) in an embedded manner; two conductive bars (2) are used for supplying power, and the third conductive bar (2) is used for controlling signals; the conductive bars (2) are electrically connected with a controller (9);
the sliding conductive assembly (3) is mounted on the conductive rail (1); the sliding conductive assembly (3) is in slidably electric connection with the conductive bars (2); and the conductive bars (2) are correspondingly electrically connected to a power supply and the controller (9);
wherein the sliding conductive assembly (3) comprises a probe control sleeve (301) and conductive probes;
the middle part of the probe control sleeve (301) is a connecting opening (304); by the connecting opening (304), the probe control sleeve (301) is capable of being mounted on the conductive rail (1); and the conductive probes (302) protrude from the connecting opening (304) and are electrically connected to the conductive bars (2) on the conductive rail (1);
wherein one side of the probe control sleeve (301) is provided with a connecting part (305) fixedly connected to a massage movement (5); and each conductive bar (2) is correspondingly electrically connected to one or more conductive probes (302);
the flexible sliding power supply assembly is **characterized in that**, the conductive rail (1) is flexible, and **in that**
the sliding conductive assembly (3) further comprises a circuit board (303) mounted in the probe control sleeve (301); the circuit board (303) is electrically connected with a plurality of conductive probes and with a micro control unit (MCU).

2. The flexible sliding power supply assembly according to claim 1, wherein one or more magnets are mounted on the conductive rail (1) in an embedded manner; a hall switch is mounted on the circuit board (303); and the hall switch is capable of determining the position of the movement by sensing a magnetic field formed by the magnets.

3. The flexible sliding power supply assembly according to claim 3, wherein the fixing slots (4) are " 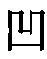 "-shaped; each fixing slot (4) comprises a fixed part (401) and an opening part (402); a width and thickness of the fixed part (401) are both equal to those of each conductive bar (2); a width of the opening part (402) is less than that of the conductive bar (2); and a thickness of the opening part (402) is less than an extending height of the conductive probe (302).

4. A massage cushion, including the flexible sliding power supply assembly according to claims 1-3, wherein the conductive rail (1) is fixedly mounted on a cushion body (8); the other side of the cushion body (8) is further provided with a rack (7); one or more massage movements (5) are mounted between the conductive rail (1) and the rack (7);
one side of each massage movement (5) is meshed with the rack (7); the other side of the massage movement (5) is fixedly provided with the sliding conductive assembly (3); and the massage movement (5) is electrically connected with each conductive bar (2) through the sliding conductive assembly (3).

5. The massage cushion according to claim 4, wherein the fixing slots (4) of the conductive rail (1) are inwardly disposed; the circuit board (303) of the probe control sleeve and one side of each conductive probe (302) is also inwardly disposed; a massage head on each massage movement (5) is outwardly disposed; and
an upper surface and a lower surface of the probe control sleeve are both of arc curved structures.

6. A control method, which is applied to the massage cushion according to claim 4 or 5, the control method specifically comprises the following steps:
S1, control: issuing, by means of a mobile phone or the controller (9), an instruction; encoding the instruction through a communication protocol; transmitting the encoded instruction to the MCU via a specified conductive bar (2); and decoding, by the MCU, the encoded instruction to control the massage movement (5) to perform a specified action; and
S2, feedback: when the massage movement (5) switches a mode or stops working, encoding, by the MCU, a state; transmitting the encoded state to the controller (9) via a specified conductive bar (2); and decoding, by a central processing unit (CPU) on the controller (9), the encoded state to obtain a working state of the massage movement (5).

7. The control method according to claim 6, further comprising step S3, positioning: sensing, by the MCU, a magnet (6) on a back shell rail of the cushion body (8) through the hall switch on the circuit board (303); after the MCU determines the position of the massage movement (5), encoding, by the MCU, position information; transmitting the encoded position information to the controller (9) via a specified conductive bar (2); and decoding, by the CPU on the controller (9), the encoded position information to obtain the position information of the massage movement (5).

## Patentansprüche

1. Flexible Schleifkontaktanordnung zur Stromversorgung, umfassend eine Leitschiene (1) und eine Schleifkontakteinheit (3), wobei die Leitschiene (1) mit drei Befestigungsnuten (4) versehen ist; wobei drei Leitstäbe (2) in den Befestigungsnuten (4) eingebettet montiert sind; wobei zwei Leitstäbe (2) zur Stromversorgung und der dritte Leitstab (2) zur Signalsteuerung dienen; wobei die Leitstäbe (2) elektrisch mit einer Steuerung (9) verbunden sind;
wobei die Schleifkontakteinheit (3) an der Leitschiene (1) montiert ist; wobei die Schleifkontakteinheit (3) in schleifender elektrischer Verbindung mit den Leitstäben (2) steht; und wobei die Leitstäbe (2) entsprechend elektrisch mit einer Stromversorgung und der Steuerung (9) verbunden sind;
wobei die Schleifkontakteinheit (3) eine Sondensteuerhülse (301) und Kontaktsonden umfasst;
wobei der mittlere Teil der Sondensteuerhülse (301) als eine Verbindungsöffnung (304) ausgebildet ist; wobei die Sondensteuerhülse (301) mittels der Verbindungsöffnung (304) an der Leitschiene (1) montierbar ist; und wobei die Kontaktsonden (302) aus der Verbindungsöffnung (304) hervorstehen und elektrisch mit den Leitstäben (2) an der Leitschiene (1) verbunden sind;
wobei eine Seite der Sondensteuerhülse (301) mit einem Verbindungsteil (305) versehen ist, das fest mit einem Massagewerk (5) verbunden ist; und wobei jeder Leitstab (2) entsprechend elektrisch mit einer oder mehreren Kontaktsonden (302) verbunden ist;
die flexible Schleifkontaktanordnung zur Stromversorgung ist **dadurch gekennzeichnet, dass** die Leitschiene (1) flexibel ist, und dass die Schleifkontakteinheit (3) ferner eine in der Sondensteuerhülse (301) montierte Leiterplatte (303) umfasst; wobei die Leiterplatte (303) elektrisch mit einer Vielzahl von Kontaktsonden und mit einer Mikrosteuereinheit (MCU) verbunden ist.

2. Flexible Schleifkontaktanordnung zur Stromversorgung nach Anspruch 1, wobei ein oder mehrere Magnete in eingebetteter Weise an der Leitschiene (1) montiert sind; wobei ein Hall-Sensor auf der Leiterplatte (303) montiert ist; und wobei der Hall-Sensor dazu eingerichtet ist, die Position des Werks durch Erfassen eines durch die Magnete gebildeten Magnetfelds zu bestimmen.

3. Flexible Schleifkontaktanordnung zur Stromversorgung nach Anspruch 3, wobei die Befestigungsnuten (4) " 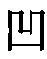 "-förmig ausgebildet sind; wobei jede Befestigungsnut (4) einen Fixierteil (401) und einen Öffnungsteil (402) umfasst; wobei eine Breite und Dicke des Fixierteils (401) jeweils denen jedes Leitstabs (2) entsprechen; wobei eine Breite des Öffnungsteils (402) geringer als die des Leitstabs (2) ist; und wobei eine Dicke des Öffnungsteils (402) geringer als eine Ausfahrhöhe der Kontaktsonde (302) ist.

4. Massagekissen, umfassend die flexible Schleifkontaktanordnung zur Stromversorgung nach den Ansprüchen 1 bis 3, wobei die Leitschiene (1) fest an einem Kissenkörper (8) montiert ist; wobei die andere Seite des Kissenkörpers (8) ferner mit einer Zahnstange (7) versehen ist; wobei ein oder mehrere Massagewerke (5) zwischen der Leitschiene (1) und der Zahnstange (7) montiert sind;
wobei eine Seite jedes Massagewerks (5) mit der Zahnstange (7) in Eingriff steht; wobei die andere Seite des Massagewerks (5) fest mit der Schleifkontakteinheit (3) versehen ist; und wobei das Massagewerk (5) über die Schleifkontakteinheit (3) elektrisch mit jedem Leitstab (2) verbunden ist.

5. Massagekissen nach Anspruch 4, wobei die Befestigungsnuten (4) der Leitschiene (1) nach innen gerichtet angeordnet sind; wobei die Leiterplatte (303) der Sondensteuerhülse und eine Seite jeder Kontaktsonde (302) ebenfalls nach innen gerichtet angeordnet sind; wobei ein Massagekopf an jedem Massagewerk (5) nach außen gerichtet angeordnet ist; und
wobei eine obere Fläche und eine untere Fläche der Sondensteuerhülse jeweils bogenförmig gekrümmte Strukturen aufweisen.

6. Steuerungsverfahren, angewendet auf das Massagekissen nach Anspruch 4 oder 5, wobei das Steuerungsverfahren spezifisch die folgenden Schritte umfasst:
S1, Steuerung: Ausgeben eines Befehls mittels eines Mobiltelefons oder der Steuerung (9); Codieren des Befehls durch ein Kommunikationsprotokoll; Übertragen des codierten Befehls an die MCU über einen spezifizierten Leitstab (2); und Decodieren des codierten Befehls durch die MCU, um das Massagewerk (5) zur Ausführung einer spezifizierten Aktion zu steuern; und
S2, Rückmeldung: wenn das Massagewerk (5) einen Modus umschaltet oder den Betrieb einstellt, Codieren eines Zustands durch die MCU; Übertragen des codierten Zustands an die Steuerung (9) über einen spezifizierten Leitstab (2); und Decodieren des codierten Zustands durch eine zentrale Verarbeitungseinheit (CPU) an der Steuerung (9), um einen Betriebszustand des Massagewerks (5) zu erhalten.

7. Steuerungsverfahren nach Anspruch 6, ferner umfassend Schritt S3, Positionierung: Erfassen eines Magnets (6) an einer Rückschalenführung des Kissenkörpers (8) durch den Hall-Sensor auf der Leiterplatte (303) mittels der MCU; nachdem die MCU die Position des Massagewerks (5) bestimmt hat, Codieren einer Positionsinformation durch die MCU; Übertragen der codierten Positionsinformation an die Steuerung (9) über einen spezifizierten Leitstab (2); und Decodieren der codierten Positionsinformation durch die CPU an der Steuerung (9), um die Positionsinformation des Massagewerks (5) zu erhalten.

## Revendications

1. Ensemble d'alimentation électrique coulissant flexible, comprenant un rail conducteur (1) et un ensemble conducteur coulissant (3), dans lequel le rail conducteur (1) est pourvu de trois rainures de fixation (4) ; trois barres conductrices (2) sont montées de manière encastrée dans lesdites rainures de fixation (4) ; deux des barres conductrices (2) sont destinées à l'alimentation électrique, et la troisième barre conductrice (2) est destinée aux signaux de commande ; les barres conductrices (2) sont électriquement connectées à un contrôleur (9) ;
l'ensemble conducteur coulissant (3) est monté sur le rail conducteur (1) ; ledit ensemble conducteur coulissant (3) est en connexion électrique coulissante avec les barres conductrices (2) ; et les barres conductrices (2) sont respectivement connectées électriquement à une source d'alimentation et audit contrôleur (9) ;
dans lequel l'ensemble conducteur coulissant (3) comprend un manchon de commande de sondes (301) et des sondes conductrices ;
la partie médiane du manchon de commande de sondes (301) est une ouverture de connexion (304) ; par l'intermédiaire de l'ouverture de connexion (304), le manchon de commande de sondes (301) est apte à être monté sur le rail conducteur (1) ; et les sondes conductrices (302) font saillie depuis ladite ouverture de connexion (304) et sont électriquement connectées aux barres conductrices (2) du rail conducteur (1) ;
dans lequel un côté du manchon de commande de sondes (301) est pourvu d'une pièce de connexion (305) fixement reliée à un mécanisme de massage (5) ; et chaque barre conductrice (2) est électriquement connectée à une ou plusieurs sondes conductrices (302) correspondantes ;
ledit ensemble d'alimentation électrique coulissant flexible étant **caractérisé en ce que** le rail conducteur (1) est flexible, et **en ce que** l'ensemble conducteur coulissant (3) comprend en outre une carte de circuit imprimé (303) montée dans le manchon de commande de sondes (301) ; ladite carte de circuit imprimé (303) est électriquement connectée à une pluralité de sondes conductrices et à une unité de commande microprogrammée (MCU).

2. Ensemble d'alimentation électrique coulissant flexible selon la revendication 1, **caractérisé en ce qu'**un ou plusieurs aimants sont montés de manière encastrée sur le rail conducteur (1) ; un interrupteur à effet Hall est monté sur la carte de circuit imprimé (303) ; et ledit interrupteur à effet Hall est apte à déterminer la position du mécanisme en détectant un champ magnétique formé par les aimants.

3. Ensemble d'alimentation électrique coulissant flexible selon la revendication 3, **caractérisé en ce que** les rainures de fixation (4) sont de forme à gorge et présentent une ouverture rétrécie ; chaque rainure de fixation (4) comprend une partie fixe (401) et une partie d'ouverture (402) ; une largeur et une épaisseur de la partie fixe (401) sont toutes deux égales à celles de chaque barre conductrice (2) ; une largeur de la partie d'ouverture (402) est inférieure à celle de la barre conductrice (2) ; et une épaisseur de la partie d'ouverture (402) est inférieure à une hauteur de saillie de la sonde conductrice (302).

4. Coussin de massage, comprenant l'ensemble d'alimentation électrique coulissant flexible selon les revendications 1 à 3, **caractérisé en ce que** le rail conducteur (1) est fixement monté sur un corps de coussin (8) ; l'autre côté du corps de coussin (8) est en outre pourvu d'une crémaillère (7) ; un ou plusieurs mécanismes de massage (5) sont montés entre le rail conducteur (1) et la crémaillère (7) ;
un côté de chaque mécanisme de massage (5) est en prise avec la crémaillère (7) ; l'autre côté du mécanisme de massage (5) est fixement pourvu de l'ensemble conducteur coulissant (3) ; et le mécanisme de massage (5) est électriquement connecté à chaque barre conductrice (2) par l'intermédiaire de l'ensemble conducteur coulissant (3).

5. Coussin de massage selon la revendication 4, **caractérisé en ce que** les rainures de fixation (4) du rail conducteur (1) sont disposées vers l'intérieur ; la carte de circuit imprimé (303) du manchon de commande de sondes et un côté de chaque sonde conductrice (302) sont également disposés vers l'intérieur ; une tête de massage sur chaque mécanisme de massage (5) est disposée vers l'extérieur ; et
une surface supérieure et une surface inférieure du manchon de commande de sondes présentent toutes deux des structures courbes en arc.

6. Procédé de commande, applicable au coussin de massage selon la revendication 4 ou 5, ledit procédé de commande comprenant spécifiquement les étapes suivantes :
S1, commande : émettre, au moyen d'un téléphone mobile ou du contrôleur (9), une instruction ; coder ladite instruction par un protocole de communication ; transmettre l'instruction codée à la MCU via une barre conductrice (2) spécifiée ; et décoder, par la MCU, l'instruction codée pour commander au mécanisme de massage (5) d'exécuter une action spécifiée ; et
S2, retour d'état : lorsque le mécanisme de massage (5) change de mode ou cesse de fonctionner, coder, par la MCU, un état ; transmettre l'état codé au contrôleur (9) via une barre conductrice (2) spécifiée ; et décoder, par une unité centrale de traitement (CPU) sur le contrôleur (9), l'état codé pour obtenir un état de fonctionnement du mécanisme de massage (5).

7. Procédé de commande selon la revendication 6, comprenant en outre l'étape S3 de positionnement : détection, par la MCU, d'un aimant (6) situé sur un rail du carter arrière du corps de coussin (8) au moyen de l'interrupteur à effet Hall sur la carte de circuit imprimé (303) ; après que la MCU a déterminé la position du mécanisme de massage (5), codage, par ladite MCU, d'informations de position ; transmission des informations de position codées au contrôleur (9) via une barre conductrice (2) spécifiée ; et décodage, par la CPU sur le contrôleur (9), des informations de position codées pour obtenir les informations de position dudit mécanisme de massage (5).
